(19) Europäisches Patentamt
European Patent Office
Office européen des brevets

(11) Veröffentlichungsnummer: **0 444 287 A1**

# (12) EUROPÄISCHE PATENTANMELDUNG

(21) Anmeldenummer: **90124681.9**

(22) Anmeldetag: **19.12.90**

(51) Int. Cl.5: **C07C 45/30**, C07C 49/403, C07C 49/385

(30) Priorität: **25.02.90 DE 4005932**

(43) Veröffentlichungstag der Anmeldung: **04.09.91 Patentblatt 91/36**

(84) Benannte Vertragsstaaten: **BE CH DE DK FR GB IT LI NL SE**

(71) Anmelder: **BAYER AG**
**W-5090 Leverkusen 1 Bayerwerk(DE)**

(72) Erfinder: **Paetz, Christian, Dr.**
**Rosenkranz 25**
**W-5093 Burscheid-Hilgen(DE)**
Erfinder: **Fiege, Helmut, Dr.**
**Walter-Flex-Strasse 23**
**W-5090 Leverkusen 1(DE)**

## (54) Verfahren zur Herstellung von Cycloalkanonen.

(57) Gegebenenfalls substituierte Cycloalkanone können hergestellt werden, indem man gegebenenfalls substituierte Cycloalkanole mit Hypohalogenit-Lösung bei pH-Werten von höchstens 6 in einem wäßrig/organischen Reaktionsmedium umsetzt.

EP 0 444 287 A1

Die Erfindung betrifft ein Verfahren zur Herstellung von Cycloalkanonen durch Umsetzung der zugehörigen Cycloalkanole mit Hypochlorit im sauren Medium.

Es ist bekannt, Cyclohexanone und alkylsubstituierte Cyclohexanone dadurch herzustellen, daß man zunächst die entsprechenden, gegebenenfalls substituierten Phenole zu den zugehörigen Cyclohexanolen hydriert und diese dann mit Schwermetallsalzen zu den gewünschten, gegebenenfalls substituierten Cyclohexanonen oxidiert; man kann auch durch partielle, zumeist in der Gasphase ausgeführte Hydrierung solcher Phenole zu einem Reaktionsgemisch kommen, in dem die gewünschten Cyclohexanon-Derivate als Hauptprodukt vertreten sind (DE-OS 34 01 343; Maslo-Zhir. Prom-st. 1987 (1), 27, zitiert nach C.A. 107 (1987), 175 524d).

Nachteilig bei solchen Verfahren ist zum einen die Verwendung von Schwermetallsalzen, wie Natrium- und Kaliumdichromat, und die damit verbundene Umweltproblematik, zum anderen die äußerst aufwendige Destillation des Reaktionsgemisches nach einer partiellen Gasphasenhydrierung, da die Siedepunkte der vorliegenden Cyclohexanol-, Cyclohexanon- und Phenol-Derivate sehr dicht beieinander liegen. Eine aufwendige Destillation ist deshalb erforderlich, weil selbst bei einer zweistufigen Hydrierung unter Wechsel des Hydrierkatalysators nur in den seltensten Fällen Gehalte von mehr als 70 % Cyclohexanon-Derivat in der Reaktionsmischung erhalten werden (DE-OS 34 01 343).

Eine weitere Möglichkeit zur Herstellung von gegebenenfalls alkylsubstituierten Cyclohexanonen besteht in der Oxidation der entsprechenden Cyclohexanole nicht mit den genannten Schwermetallen (Salze von Chrom, Blei und Selen oder der relativ teuren Permanganate), sondern mit Hypohalogeniten und N-Halogen-Verbindungen (Houben-Weyl, Band VII/2a (1973), S. 699ff., besonders S. 758-763). Bei diesen zuletzt genannten Verfahren werden die Oxidationsreaktionen jeweils im alkalischen Milieu durchgeführt, denn es wird in der genannten Literaturstelle auf S. 758 ausdrücklich darauf verwiesen, daß freies Halogen, wie es aus Hypohalogeniten und N-Halogen-Verbindungen im sauren Bereich entsteht, für die Oxidation von insbesondere cyclischen sekundären Alkoholen nicht geeignet ist, weil der Eintritt von Halogen in das Ringsystem eine sehr leicht erfolgende Nebenreaktion ist. Deshalb wird auch dort, wo vermeintlich mit freiem Halogen gearbeitet wird, im alkalischen Bereich gearbeitet, so daß das sich bildende Hypohalogenit das eigentlich oxidierende Agens ist. Die Ausbeuten und die Reinheiten der Reaktionsprodukte bei diesen zuletzt genannten Verfahren sind jedoch völlig unbefriedigend und daher für eine technische Durchführung nicht geeignet.

Es ist auch bereits versucht worden, Carbonylverbindungen, wie 1,4-Cyclohexandion, durch Oxidation von 1,4-Cyclohexandiol mit Natriumhypochlorit in Gegenwart von Salzsäure bei einem pH-Wert von 1 bis 2 zu erhalten. Hierbei wird im wäßrigen Medium in Gegenwart von Salzsäure und in weiterer Gegenwart eines Katalysators aus der Gruppe der Platinmetalle, beispielsweise $RuCl_3$, in einer Menge von 2 Mol-%, bezogen auf den Dialkohol, gearbeitet (JP 01/50 836, zitiert nach C.A. 111 (1989), 57 115f; JP 63/174 946, zitiert nach C.A. 109 (1988), 230 388f).

Der Nachteil bei dem zuletzt genannten Verfahren ist der Verbrauch von sehr teurem Rutheniumchlorid in relativ großer Menge, welches nach der Reaktion nur sehr schwer aus der Reaktionsmischung, beispielsweise durch Fällung, abzutrennen ist und daher nicht direkt zurückgeführt werden kann. Weiterhin ist bekannt, daß die im sauren Medium aus Hypochloriten freigesetzte unterchlorige Säure mit ihrem Anhydrid Dichloroxid im Gleichgewicht steht. Dichloroxid ist jedoch eine endotherme Verbindung, die explosionsartig in ihre Bestandteile zerfallen kann (Lehrbücher der anorganischen Chemie).

Wasserstoffperoxid ist als Oxidationsmittel für sekundäre Alkohole zur Gewinnung der zugehörigen Ketone nicht geeignet.

Es wurde nun gefunden, daß man Cycloalkanole mit Alkali- oder Erdalkali-Hypohalogeniten in einem sauren Reaktionsmedium in glatter Reaktion zu den zugehörigen Cycloalkanonen oxidieren kann, wenn das Reaktionsmedium organisch/wäßrig ist.

Es wurde ein Verfahren zur Herstellung von Cycloalkanonen mit 3 bis 8 Ring-C-Atomen, die an einem oder mehreren C-Atomen ein- oder zweifach substituiert sein können, gefunden, das dadurch gekennzeichnet ist, daß man Cycloalkanole der Formel

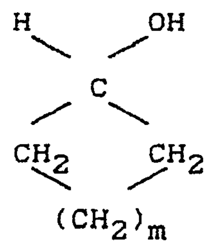

(I),

worin

m eine ganze Zahl von null bis fünf bedeutet und worin

ein oder mehrere C-Atome unabhängig voneinander ein- oder zweifach durch geradkettiges oder verzweigtes $C_1$-$C_{12}$-Alkyl, geradkettiges oder verzweigtes $C_1$-$C_{12}$-Alkoxy, Fluor, Chlor oder Brom substituiert sein können, wobei die Zahl der Substituenten höchstens 5 beträgt,

in einem organisch/wäßrigen Reaktionsmedium, das aus Wasser und einem mit Wasser mischbaren organischen Lösungsmittel besteht, mit Alkali- oder Erdalkalihypohalogeniten bei einem pH-Wert, der höchstens 6 ist, umsetzt.

Geradkettiges oder verzweigtes Alkyl ist beispielsweise Methyl, Ethyl, Propyl, Isopropyl, Butyl, Isobutyl, tert.-Butyl, die isomeren Pentyle, Hexyle, Octyle, Decyle oder Dodecyle sowie der cyclische Alkylrest Cyclopentyl oder Cyclohexyl. Alkyl hat in bevorzugter Weise 1 bis 8 C-Atome und ist besonders bevorzugt Methyl, tert.-Butyl oder tert.-Octyl.

Geradkettiges oder verzweigtes Alkoxy ist Methoxy, Ethoxy, Propoxy, Isopropoxy, Butoxy, Isobutoxy, tert.-Butoxy, die isomeren Pentyloxy, Hexyloxy, Octyloxy, Decyloxy oder Dodecyloxy. Bevorzugtes Alkoxy hat 1 bis 4 C-Atome, besonders bevorzugtes Alkoxy hat 1 oder 2 C-Atome.

In bevorzugter Weise werden erfindungsgemäß Cyclohexanole der Formel

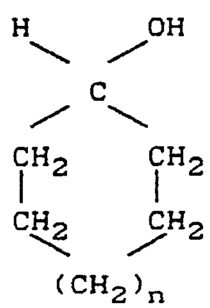

(II)

umgesetzt, worin

n die Zahl null oder eins bedeutet und worin

ein oder mehrere C-Atome unabhängig voneiander ein- oder zweifach durch geradkettiges oder verzweigtes $C_1$-$C_{12}$-Alkyl, geradkettiges oder verzweigtes $C_1$-$C_{12}$-Alkoxy, Fluor, Chlor oder Brom substituiert sein können und wobei die Zahl der Substituenten höchstens 5 beträgt.

In besonders bevorzugter Weise werden im erfindungsgemäßen Verfahren Cyclohexanole umgesetzt, worin ein oder mehrere C-Atome unabhängig voneinander ein- oder zweifach durch geradkettiges oder verzweigtes $C_1$-$C_{12}$-Alkyl, geradkettiges oder verzweigtes $C_1$-$C_{12}$-Alkoxy, Fluor, Chlor oder Brom substituiert sein können und wobei die Zahl der Substituenten höchstens 5 beträgt.

Der Index m im Rahmen der Formel (I) bedeutet eine ganze Zahl von null bis sechs, so daß Formel (I) ein Derivat von Cyclopropanol, Cyclobutanol, Cyclopentanol, Cyclohexanol, Cycloheptanol bzw. Cyclooctanol umfaßt. Der Index n in der Formel (II) bedeutet die Zahl null oder eins, so daß Formel (II) ein Derivat von Cyclopentanol bzw. Cyclohexanol umfaßt. Ein oder mehrere C-Atome des Ringgerüstes können in der oben beschriebenen Weise substituiert sein. Wie es bei cycloaliphatischen Systemen häufig anzutreffen ist, können C-Atome des Ringsystems auch doppelt substituiert sein; in vielen Fällen erfolgt diese doppelte Substitution durch zwei gleiche Reste (geminale Substitution). Ansonsten können die Substituenten an verschiedenen C-Atomen unterschiedlich sein. In einleuchtender Weise ist die Zahl der möglichen Substituenten beim Cyclopropanol auf 4, in den anderen Fällen auf 5 begrenzt. Es ist schließlich nicht erforderlich, daß die erfindungsgemäß umzusetzenden cyclischen Substrate überhaupt substituiert sind; in diesem Falle

handelt es sich um die nicht substituierten Cycloalkanole.

In bevorzugter Weise werden Cycloalkanole umgesetzt, bei denen die Zahl der Substituenten höchstens 3, bevorzugt höchstens 2, beträgt. Weiterhin wichtig sind Cycloalkanole, bei denen der Bedeutungsumfang der Substituenten auf $C_1$-$C_{12}$-Alkyl, bevorzugt $C_1$-$C_8$-Alkyl, der obengenannten Art beschränkt ist. Besonders wichtig ist das erfindungsgemäße Verfahren für Cycloalkanole, die mindestens einen solcher Substituenten tragen.

Das erfindungsgemäße Verfahren wird in einem organisch/ wäßrigen Reaktionsmedium vorgenommen, dessen organische Komponente mit Wasser mischbar ist. Als organische Komponente kommt ein Alkohol, ein Keton, ein mit Wasser mischbarer Ether oder eine Carbonsäure in Frage. Beispiele hierfür sind Methanol, Ethanol, Propanol, Isopropanol, Butanol, tert.-Butanol, Aceton, Methyl-ethylketon, Dibutylketon, Tetrahydrofuran, Dioxan, Essigsäure, Propionsäure, Buttersäure. In bevorzugter Weise wird ein Alkohol oder eine Carbonsäure der genannten Art eingesetzt. Besonders bevorzugte organische Komponenten für das erfindungsgemäße Verfahren sind Methanol, Ethanol oder Essigsäure. Es kann auch ein Gemisch mehrerer dieser organischen Komponenten eingesetzt werden. Da auch die umzusetzenden Cycloalkanole in Abhängigkeit von ihrem Substitutionsmuster mit Wasser mischbare Alkohole darstellen, kann bei ausreichender Wassermischbarkeit solcher Cycloalkanole auf die Mitverwendung weiterer organischer Komponenten für das Reaktionsmedium verzichtet werden. Die Menge der organischen Komponente wird so bemessen, daß ein im wesentlichen homogenes Reaktionsgemisch entsteht, so daß beispielsweise die Rührfähigkeit des Reaktionsgemisches gewährleistet ist. Typische Mengen des Reaktionsmediums insgesamt (Wasser und organische Komponente) sind 0,4-4 kg, bevorzugt 0,6-2 kg Reaktionsmedium pro 100 g Cycloalkanol.

Die Menge des Wassers und der organischen Komponente stehen hierbei in einem Gewichtsverhältnis von 0,5-2:1, bevorzugt nahe 1:1.

Der Anteil der organischen Komponente beträgt, bezogen auf 100 g des umzusetzenden Cycloalkanols, 0,2-2,6 kg.

Das Oxidationsmittel ist ein Alkali- oder Erdalkalihypohalogenit, beispielsweise Natriumhypochlorit, Natriumhypobromit, Kaliumhypochlorit, Kaliumhypobromit, Calciumhypochlorit, Calciumhypobromit. In bevorzugter Weise wird ein Alkalihypohalogenit, wie Natrium- oder Kaliumhypochlorit oder -bromit eingesetzt. In besonders bevorzugter Weise wird Natriumhypochlorit verwendet. Das Hypohalogenit kann in reiner Form oder in Form einer wäßrigen Lösung eingesetzt werden. Diese Lösung kann auch in Form einer technischen Bleichlauge eingesetzt werden. Weiterhin ist es möglich, das Hypohalogenit "in situ" zu erzeugen. Hierzu leitet man in das organisch/ wäßrige Reaktiosmedium, das neben dem umzusetzenden gegebenenfalls substituierten Cycloalkanol noch einen Puffer, wie einen Phosphatpuffer, enthält, der im gewünschten pH-Bereich wirksam ist, Halogen, beispielsweise Chlor, ein. Durch diese Einleitung von Halogen wird im Reaktionsgemisch in situ Hypohalogenit bzw. die freie unterhalogenige Säure gebildet, durch die dann die oxidative Umsetzung zum gegebenenfalls substituierten Cycloalkanon erfolgt.

Das Hypohalogenit wird in einer Menge von 70 bis 250 % der dem Fachmann bekannten Stöchiometrie, bezogen auf die Cyclohexanole, bevorzugt in einer Menge von 100 bis 150 % der Stöchiometrie, eingesetzt.

Der pH-Wert für die erfindungsgemäße Umsetzung beträgt höchstens 6, beispielsweise 1 bis 6; der bevorzugte pH-Wert beträgt 2 bis 4,5.

Zur Einstellung des gewünschten pH-Wertes kann eine Säure oder eine sauer wirkende Verbindung eingesetzt werden, beispielsweise anorganische Säuren, wie Salzsäure, Schwefelsäure, Phosphorsäure, sauer reagierende anorganische Salz, wie Hydrogensulfat oder primäre Phosphate, oder eine mit Wasser mischbare organische Säure, wie Ameisensäure, Essigsäure, Propionsäure oder Buttersäure. Der Einsatz einer anorganischen oder einer mit Wasser mischbaren organischen Säure ist bevorzugt. Die genannten mit Wasser mischbaren organischen Säuren können gleichzeitig auch die organische Komponente oder einen Teil der organischen Komponente für das organisch/wäßrige Reaktionsmedium darstellen. Unter ihnen sind Essigsäure und Propionsäure, besonders Essigsäure, bevorzugt.

Das erfindungsgemäße Verfahren kann bei einer Temperatur von -20 bis +80 °C, bevorzugt von 0 bis 50 °C, durchgeführt werden.

Das erfindungsgemäße Verfahren kann sowohl diskontinuierlich als auch kontinuierlich durchgeführt werden.

Im erfindungsgemäßen Verfahren werden Cycloalkanone der Formel

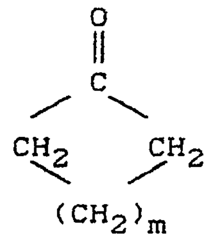 (III),

erhalten, worin

m eine ganze Zahl von null bis fünf bedeutet und worin

ein oder mehrere C-Atome unabhängig voneinander ein- oder zweifach durch geradkettiges oder verzweigtes $C_1$-$C_{12}$-Alkyl, geradkettiges oder verzweigtes $C_1$-$C_{12}$-Alkoxy, Fluor, Chlor oder Brom substituiert sein können, wobei die Zahl der Substituenten höchstens 5 beträgt.

Beispielhaft läßt sich das erfindungsgemäße Verfahren durch das folgende Formelschema darstellen:

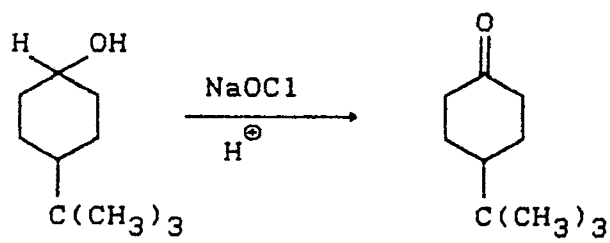


Die erfindungsgemäß einsetzbaren Cycloalkanole der Formel (I) sind dem Fachmann bekannt. Das besonders wichtige Cyclohexanol und seine Substitutionsderivate können beispielsweise leicht durch Hydrierung von Phenol bzw. substituierten Phenolen hergestellt werden.

Die erfindungsgemäß erhältlichen, gegebenenfalls substituierten, Cycloalkanone fallen in hoher Reinheit und mit hohen Ausbeuten an.

Das erfindungsgemäße Verfahren vermeidet in wirtschaftlich vorteilhafter Weise den Einsatz umweltproblematischer Oxidationsmittel und teurer Katalysatoren. Weiterhin ist die erfindungsgemäße Arbeitsweise ohne den Einsatz von Spezialapparaturen durchführbar.

Die Durchführung des erfindungsgemäßen Verfahrens gelingt überraschenderweise gegen die weiter oben dargestellte herrschende Lehrmeinung in glatter Weise. Es ist weiterhin überraschend, daß eine Oxidation substituierter Cycloalkanole, beispielsweise der alkylsubstituierten Cycloalkanole mit Alkalihypohalogenit unter den allgemein bekannten alkalischen Reaktionsbedingungen keinen Umsatz ergibt; diese Reaktion kann wiederum nur unter Einsatz der teuren und nicht wiederverwendbaren Edelmetallsalze, wie Rutheniumchlorid, in Gang gesetzt werden.

Die nach dem erfindungsgemäßen Verfahren erhaltenen, gegebenenfalls substituierten Cycloalkanone sind Zwischenprodukte, beispielsweise bei der Herstellung von Pflanzenschutzmitteln. Weiterhin können sie als Keton-Baustein bei der Umsetzung mit Phenolen zu Bisphenol-Derivaten dienen, die ihrerseits wiederum Monomer-Bausteine für Polycarbonate oder Polyester darstellen; spezielle Bisphenole unter Verwendung erfindungsgemäß hergestellter Cycloalkanone können weiterhin beispielsweise zur Herstellung von LC-(Liquid Crystals)-Polymeren dienen.

Beispiel 1

Man löste 202 g (1,27 Mol) 4-tert.-Butylcyclohexanol in 800 ml Methanol, stellte diese Lösung durch einige Tropfen 10 %iger Schwefelsäure auf pH 4 und tropfte danach unter Kühlen auf 25° C innerhalb von 1 h 880 g Natriumhypochloritlösung mit 13 % aktivem Chlor = 1,6 Mol NaOCl hinzu; gleichzeitig tropfte man unter Kontrolle einer pH-Elektrode soviel 10 %ige Schwefelsäure synchron hinzu, daß der pH-Wert 4 ± 1 während der Reaktion eingehalten wurde. Dazu wurden 198 ml 10 %ige Schwefelsäure benötigt. Anschließend wurde noch 1 h bei pH 4 nachgerührt, danach wurde unter atmosphärischem Druck bis zu einer Sumpftemperatur von 100° C das Methanol abdestilliert und durch 300 ml Toluol ersetzt. Nach Abkühlen des 2-Phasen-Systems und Abtrennen der organischen Phase wurde die wäßrige Phase nochmals mit 200

ml Toluol extrahiert. Danach wurden die toluolischen Phasen vereinigt und andestilliert. Diese toluolische Lösung des 4-tert.-Butylcyclohexanons konnte nun für weitere Reatkionen direkt eingesetzt werden. Nach vollständiger Entfernung des Lösungsmittels erhielt man 199,5 g 4-tert.-Butylcyclohexanon = 96 % Ausbeute mit einer Reinheit von 96 %.

Beispiel 2

Man legte eine Lösung von 39 g (0,25 Mol) 4-tert.-Butylcyclohexanol in 250 ml Methanol vor, gab dazu eine Lösung von 100 g $NaH_2PO_4$ in 100 ml Wasser und tropfte dann bei 40° C innerhalb von 1 h 165 g (0,3 Mol) NaOCl-Lösung mit 13 % aktivem Chlor zu diesem Reaktionsgemisch hinzu. Der pH-Wert lag zu Beginn der Reaktion bei 4,5, bei Reaktionsende bei pH 3,8. Man rührte dann noch 1 h bei der angegebenen Temperatur nach; die Aufarbeitung erfolgte wie im Beispiel 1. Man erhielt 37,2 g Rohprodukt = 92,8 % der Theorie mit einer Reinheit von 96,6 %.

Beispiel 3

Man legte eine Lösung von 39 g (0,25 Mol) 4-tert.-Butylcyclohexanol in 250 ml Essigsäure vor und tropfte bei 15° C innerhalb von 1 h 165 g (0,3 Mol) NaOCl-Lösung mit 13 % aktivem Chlor zu dieser Lösung hinzu. Dabei wurde die ehemals homogene Lösung leicht trüb. Man rührte dann noch 1 h bei der angegebenen Temperatur nach und goß dann den Reaktionsansatz auf 1 l Wasser. Man extrahierte die wäßrige Phase 3 x mit 200 ml Toluol, wusch danach die organische Phase neutral und dampfte anschließend das Lösungsmittel ab. Man erhielt 34,9 g Rohprodukt = 91 % der Theorie mit einer Reinheit von 93 %.

Beispiel 4

Man hydrierte in einem VA-Autoklaven bei 150° C in Gegenwart von Raney-Nickel und 150 bar $H_2$ eine Lösung aus 225 g 4-tert.-Butylphenol in 300 ml Methanol. Nach Öffnen des Autoklaven verdünnte man mit 650 ml Methanol und filtrierte anschließend vom Hydrierkontakt ab. In den danach resultierenden ca. 1150 ml Lösung waren 228,7 g 4-tert.-Butylcyclohexanol in einer Reinheit von 98,7 % enthalten.

Für die nachfolgende Oxidation mit Natriumhypochloritlösung wurden von diesen 1150 ml Lösung 1000 ml eingesetzt; diese 1000 ml enthielten 202 g 4-tert.-Butylcyclohexanol (98 %ig) = 1,27 Mol.

Zu dieser Lösung, die mit einigen Tropfen 10 %iger Schwefelsäure auf pH 4 gebracht worden war, ließ man bei 25° C unter Kühlen innerhalb 1 h 880 g Natriumhypochloritlösung mit 13 % aktivem Chlor = 1,6 Mol NaOCl hinzutropfen; gleichzeitig tropfte man unter Kontrolle mit einer pH-Elektrode soviel 10 %ige Schwefelsäure synchron hinzu, daß der pH-Wert 4 ± 1 eingehalten wurde. Dazu wurden ca. 198 ml 10 %ige Schwefelsäure benötigt.

Nach vollendeter Dosierung wurde noch 1 h bei pH 4 nachgerührt, danach aus dem Reaktionsgemisch das Methanol unter atmosphärischem Druck bis zu einer Sumpftemperatur von 100° C abdestilliert. Anschließend gab man zu dem mittlerweile auf 80° C abgekühlten Gemisch 300 ml Toluol, kühlte weiter ab, trennte die organische Phase ab und extrahierte die wäßrige Phase nochmals mit 200 ml Toluol. Danach wurden die toluolischen Phasen vereinigt und andestilliert. Für folgende Umsetzungen konnte nun entweder die toluolische Lösung, die 199,5 g = 96 % der Theorie 4-tert.-Butylcyclohexanon in einer Reinheit von 96 % enthielt, verwendet werden, oder man erhielt nach Eindampfen des Lösungsmittels die reine Verbindung vom Schmelzpunkt 47 bis 48° C.

Beispiel 5

Man legte eine Lösung von 32 g (0,25 Mol) 4-tert.-Butylcyclohexanol in 250 ml Methanol vor, gab dazu eine Lösung von 100 g $NaH_2PO_4$ in 100 ml Wasser und leitete dann bei 25° C innerhalb 2 h 18 g (0,25 Mol) Chlor in die Reaktionsmischung ein (Oxidation mit in situ erzeugter Chlorlauge). Der pH-Wert des Reaktionsgemisches sank von einem Anfangswert von pH 5,1 auf einen Wert von pH 1,6 nach Beendigung der Chloreinleitung. Die Aufarbeitung erfolgte wie im Beispiel 1 sofort im Anschluß an die Beendigung der Chloreinleitung. Man erhielt 38,8 g Rohprodukt (= 100 % der theoretischen Menge) das zu 66 % aus 4-tert.-Butylcyclohexanon und zu 26 % aus Einsatzmaterial besteht; der Rest bestand aus 9 nicht identifizierten Verbindungen.

Beispiel 6

Setzte man in Beispiel 2 bei sonst gleichen Reaktionsbedingungen statt Methanol nunmehr Ethanol ein, so erhielt man 36,1 g Rohprodukt (= 94 % der theoretischen Menge) das zu 78 % aus 4-tert.-Butylcyclohexanon und zu 20 % aus Einsatzmaterial besteht.

Beispiel 7

Setzte man in Beispiel 2 unter sonst gleichen Reaktionsbedingungen statt 0,3 Mol NaOCl-Lösung nur die stöchiometrische Menge von 0,25 Mol ein, so erhielt man in 93 %iger Ausbeute ein Rohprodukt, das zu 85 % aus 4-tert.-Butylcyclohexanon und zu 12 % aus Einsatzmaterial besteht. Der Rest bestand aus nicht identifizierten Verbindungen.

Beispiel 8

Setzte man in Beispiel 2 unter sonst gleichen Reaktionsbedingungen statt 4-tert.-Butylcyclohexanol nunmehr 28,5 g (0,25 Mol) 3-Methylcyclohexanol - hergestellt durch Hydrierung von m-Kresol - ein, so erhielt man 10,2 g Rohprodukt (= 37 % der theoretischen Menge) einer 79 %igen Ware, die noch 6 % Einsatzmaterial enthielt.

Beispiel 9

Setzte man in Beispiel 2 unter sonst gleichen Reaktionsbedingungen statt 4-tert.-Butylcyclohexanol nunmehr 53 g (0,25 Mol) 4-tert.-Octylcyclohexanol - hergestellt durch Hydrierung von 4-tert.-Octylphenol - ein, so erhielt man 50,4 g Rohprodukt (= 96 % der theoretischen Menge) einer 90 %igen Ware, die noch 2 % Einsatzmaterial enthielt.

Beispiel 10

Setzte man in Beispiel 2 unter sonst gleichen Reaktionsbedingungen statt 4-tert.-Butylcyclohexanol nunmehr 35,5 g (0,25 Mol) 3,3,5-Trimethylcyclohexanol ein, so erhielt man 33,9 g Rohprodukt (= 96 % der theoretischen Menge) einer 83 %igen Ware.

Beispiel 11

Setzte man in Beispiel 2 unter sonst gleichen Reaktionsbedingungen statt 4-tert.-Butylcyclohexanol nunmehr 32 g (0,25 Mol) 3,5-Dimethylcyclohexanol ein, so erhielt man 16,7 g Rohprodukt (= 53 % der theoretischen Menge) einer 75 %igen Ware.

## Patentansprüche

1. Verfahren zur Herstellung von Cycloalkanonen mit 3 bis 8 Ring-C-Atomen, die an einem oder mehreren C-Atomen ein- oder zweifach substituiert sein können, dadurch gekennzeichnet, daß man Cycloalkanole der Formel

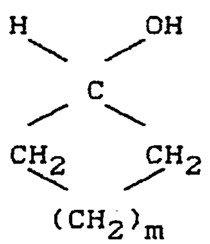

(I),

worin

m eine ganze Zahl von null bis fünf bedeutet und worin

ein oder mehrere C-Atome unabhängig voneinander ein-oder zweifach durch geradkettiges oder verzweigtes $C_1$-$C_{12}$-Alkyl, geradkettiges oder verzweigtes $C_1$-$C_{12}$-Alkoxy, Fluor, Chlor oder Brom substituiert sein können, wobei die Zahl der Substituenten höchstens 5 beträgt,

in einem organisch/wäßrigen Reaktionsmedium, das aus Wasser und einem mit Wasser mischbaren organischen Lösungsmittel besteht, mit Alkali- oder Erdalkalihypohalogeniten bei einem pH-Wert, der höchstens 6 ist, umsetzt.

**2.** Verfahren nach Anspruch 1, dadurch gekennzeichnet, daß man ein Cycloalkanol der Formel

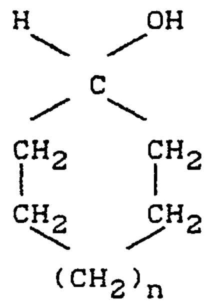

umsetzt, worin
n die Zahl null oder eins bedeutet und worin
ein oder mehrere C-Atome unabhängig voneinander ein- oder zweifach durch geradkettiges oder verzweigtes $C_1$-$C_{12}$-Alkyl, geradkettiges oder verzweigtes $C_1$-$C_{12}$-Alkoxy, Fluor, Chlor oder Brom substituiert sein können, wobei die Zahl der Substituenten höchstens 5 beträgt.

**3.** Verfahren nach Anspruch 2, dadurch gekennzeichnet, daß man ein Cyclohexanol umsetzt, wobei ein oder mehrere C-Atome unabhängig voneinander ein- oder zweifach durch geradkettiges oder verzweigtes $C_1$-$C_{12}$-Alkyl, geradkettiges oder verzweigtes $C_1$-$C_{12}$-Alkoxy, Fluor, Chlor oder Bom substituiert sein können und wobei die Zahl der Substituenten höchstens 5 beträgt.

**4.** Verfahren nach Anspruch 1, dadurch gekennzeichnet, daß man als Hyophalogenit Natrium- oder Kaliumhypohalogenit, bevorzugt Natrium- oder Kaliumhypochlorit oder -bromit, besonders bevorzugt Natriumhypochlorit, einsetzt.

**5.** Verfahren nach Anspruch 1, dadurch gekennzeichnet, daß man das Hypochlorit in 70 bis 250 % der Stöchiometrie, bezogen auf das Cycloalkanol, bevorzugt 100 bis 150 % der Stöchiometrie, einsetzt.

**6.** Verfahren nach Anspruch 1, dadurch gekennzeichnet, daß die Reaktionstemperatur -20 °C bis +80 °C, bevorzugt 0 bis 50 °C, beträgt.

**7.** Verfahren nach Anspruch 1, dadurch gekennzeichnet, daß das mit Wasser mischbare Lösungsmitttel ein Alkohol, Keton, Ether oder eine Carbonsäure ist, bevorzugt ein Alkohol oder eine Carbonsäure ist und besonders bevorzugt Methanol, Ethanol oder Essigsäure ist.

**8.** Verfahren nach Anspruch 1, dadurch gekennzeichnet, daß die zur pH-Wert-Einstellung eingesetzte Säure eine anorganische Säure oder eine mit Wasser mischbare organische Säure darstellt.

**9.** Verfahren nach Anspruch 1, dadurch gekennzeichnet, daß der pH-Wert bei der Umsetzung im Bereich von 1 bis 6, bevorzugt im Bereich 2 bis 4,5, gehalten wird.

Europäisches Patentamt

# EUROPÄISCHER RECHERCHENBERICHT

Nummer der Anmeldung

**EP 90 12 4681**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | PATENT ABSTRACTS OF JAPAN Band 12, Nr. 405 (C-539)(3252), 26. Oktober 1988; & JP - A - 63145248 (MITSUBISHI CHEM.) 17.06.1988 * das ganze Dokument * --- | 1-6,8,9 | C 07 C 45/30<br>C 07 C 49/403<br>C 07 C 49/385 |
| X | JOURNAL OF ORGANIC CHEMISTRY Band 45, Nr. 10, 9. Mai 1980, Seiten 2030-2032, American Chem. Soc., Columbus, US; R.V. STEVENS et al.: "Convenient and inexpensive procedure for oxidation of secondary alcohols to ketones" * Seiten 2031,2032; Tabelle 1; Beispiele * --- | 1-9 | |
| X | CHEMICAL ABSTRACTS Band 106, Nr. 1, 5. Januar 1987, Seite 422, Zusammenfassung Nr. 4497x, Columbus, Ohio, US; Y. BLAZAT et al.: "Oxidation of secondary alcohols using Javelle water" & Bull. Union Physicians 1985, Band 80, Nr. 678, Seiten 239-243 * Zusammenfassung * --- | 1-9 | |
| A | CHEMICAL ABSTRACTS Band 103, Nr. 9, 2. September 1985, Seite 586, Zusammenfassung Nr. 70538a, Columbus, Ohio, US; J.R. MOHRING et al.: "The design of laboratory experiments in the 1980's: a case study on the oxidation of alcohols with household bleach" & J. Chem. Educ. 1985, Band 62, Nr. 6, Seiten 519-521 * Zusammenfassung * --- | 1 | **RECHERCHIERTE SACHGEBIETE (Int. Cl.5)**<br>C 07 C |
| A | PATENT ABSTRACTS OF JAPAN Band 1, Nr. 45 (C-1803)(76), 4. Mai 1977; & JP - A - 523014 (MITSUBISHI K.K.) 01.11.1977 * das ganze Dokument * --- | 1 | |

-/-

Der vorliegende Recherchenbericht wurde für alle Patentansprüche erstellt

| Recherchenort | Abschlußdatum der Recherche | Prüfer |
|---|---|---|
| Berlin | 29 Mai 91 | PROBERT C.L. |

KATEGORIE DER GENANNTEN DOKUMENTE
X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus anderen Gründen angeführtes Dokument
&: Mitglied der gleichen Patentfamilie, übereinstimmendes Dokument

Europäisches Patentamt

# EUROPÄISCHER RECHERCHENBERICHT

Nummer der Anmeldung

**EP 90 12 4681**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | GB-A-2 119 362 (CIBA GEIGY)<br>* Seite 2, Zeilen 50-59 *<br>- - - - - | 1 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| Berlin | 29 Mai 91 | PROBERT C.L. |

KATEGORIE DER GENANNTEN DOKUMENTE
- X: von besonderer Bedeutung allein betrachtet
- Y: von besonderer Bedeutung in Verbindung mit einer anderen Veröffentlichung derselben Kategorie
- A: technologischer Hintergrund
- O: nichtschriftliche Offenbarung
- P: Zwischenliteratur
- T: der Erfindung zugrunde liegende Theorien oder Grundsätze
- E: älteres Patentdokument, das jedoch erst am oder nach dem Anmeldedatum veröffentlicht worden ist
- D: in der Anmeldung angeführtes Dokument
- L: aus anderen Gründen angeführtes Dokument
- &: Mitglied der gleichen Patentfamilie, übereinstimmendes Dokument